Europäisches Patentamt

European Patent Office

Office européen des brevets

(19)

(11) Veröffentlichungsnummer: **0 031 022**
**B1**

(12) **EUROPÄISCHE PATENTSCHRIFT**

(45) Veröffentlichungstag der Patentschrift:
25.05.83

(21) Anmeldenummer: 80106967.5

(22) Anmeldetag: 12.11.80

(51) Int. Cl.³: **F 16 L 31/00, F 16 L 33/00,
A 61 M 5/00, A 61 M 25/00**

(54) Schlauchverbindung für medizinische Geräte.

(30) Priorität: 12.12.79 DE 2949865

(43) Veröffentlichungstag der Anmeldung:
01.07.81 Patentblatt 81/26

(45) Bekanntmachung des Hinweises auf die Patenterteilung:
25.05.83 Patentblatt 83/21

(84) Benannte Vertragsstaaten:
AT BE CH DE FR GB IT LI LU NL SE

(56) Entgegenhaltungen:
DE-A-2 718 956
DE-B-1 766 739
FR-A-2 327 483
US-A-3 768 476
US-A-4 045 058
US-A-4 068 870

(73) Patentinhaber: Intermedicat GmbH, Gerliswilstrasse 45,
CH-6020 Emmenbrücke (CH)

(72) Erfinder: Voges, Karl-Friedrich, Lindenbergstrasse 18,
D-3508 Melsungen (DE)
Erfinder: Haacke, Claus, Dr., Lindenbergstrasse 6,
D-3508 Melsungen (DE)
Erfinder: Heise, Peter, Heiligenbergstrasse 23,
D-3501 Fuldabrück 3 (DE)

(74) Vertreter: von Kreisler, Alek, Dipl.-Chem. et al,
Deichmannhaus am Hauptbahnhof, D-5000 Köln 1 (DE)

BUNDESDRUCKEREI BERLIN

## Schlauchverbindung für medizinische Geräte

Die Erfindung betrifft eine Schlauchverbindung für medizinische Geräte, mit einem an dem einen Schlauchende befestigten ersten Endstück und einem an dem zweiten Schlauchende befestigten zweiten Endstück, das mit dem ersten Endstück durch Einschieben abdichtend verbindbar ist, und einer an einem der Endstücke angreifenden Halterung, die über mindestens eine längslaufende Lasche das andere Endstück festhält.

Es ist bekannt, die Schläuche von Kathetern od. dgl. mit anderen Schläuchen dadurch zu verbinden, daß der eine Schlauch ein Endstück mit einem starren Außenkegel und der andere Schlauch ein Endstück mit einem flexiblen Innenkegel erhält. Der Außenkegel des einen Endstückes wird in den Innenkegel des anderen Endstückes eingedrückt. Da solche Schlauchverbindungen nicht genormt sind, weichen die Durchmesser, Längen und Kegelwinkel der Endstücke von Hersteller zu Hersteller ab. Infolge der Elastizität des Innenkegels können dennoch Schlauchenden mit abweichenden Kegelmaßen benutzt werden. Dabei ist jedoch die Gefahr des unbeabsichtigten Lösens besonders groß.

Ein Anwendungsgebiet von Schlauchverbindungen, bei dem die Sicherheit gegen akzidentelle Diskonnektion besonders wichtig ist, ist die Verbindung von Blasenkathetern mit Harnsammelsystemen. Bei unruhigen oder unvorsichtigen Patienten kommt es durch Zugbelastung der Schläuche häufig zu einem unbeabsichtigten Lösen der Verbindung, so daß Urin austritt und die Verbindungsstelle mit Bakterien kontaminiert wird. Untersuchungen haben gezeigt, daß Kontaminationen der Verbindungsstelle ein Hauptausgangspunkt für aufsteigende Harnweginfektion ist. Bei besonderer Kontaminationsgefahr darf daher die Schlauchverbindung überhaupt nicht — auch nicht durch das Pflegepersonal — zu lösen sein.

Eine bekannte Schlauchverbindung der eingangs genannten Art (US-A-4 068 870) weist zwei ineinandersteckbare zylindrische Endstücke auf, die einander angepaßt sind und abdichtend ineinandergreifen. Zur Sicherung der Schlauchverbindung ist an dem einen Endstück eine schwenkbare Verriegelungsvorrichtung angebracht, die im Verriegelungszustand eine Schulter des anderen Endstückes hintergreift, das Auseinanderziehen der Endstücke zu verhindern. Eine derartige Schlauchverbindung benötigt zwei in ihren Formen und Abmessungen genau aufeinander abgestimmte Endstücke.

Bei einer anderen bekannten Schlauchverbindung der eingangs genannten Art (DE-B-1 766 739) sind ebenfalls zwei zueinander passende und abdichtend ineinanderschiebbare zylindrische Endstücke vorgesehen, die mit achsparallelen Laschen gegen Auseinanderziehen gesichert werden. Auch diese Verbindungstechnik setzt ganz bestimmte Endstücke voraus, die ineinanderpassen. Diese Voraussetzung ist bei medizinischen Geräten jedoch nicht immer gegeben.

Bekannt ist schließlich auch eine Schlauchverbindung mit ineinandergreifenden konischen Endstücken (FR-A1-2 327 483). Bei dieser Verbindungsvorrichtung sind die Endstücke jedoch mit Schraubteilen ausgestattet, durch die die konischen Abschnitte durch gegenseitiges Verdrehen ineinandergedrückt werden. Auch bei dieser Konstruktion müssen die Endstücke genau aufeinander abgestimmt sein, um eine Abdichtung und einen festen Halt der Schlauchverbindung zu gewährleisten.

Der Erfindung liegt die Aufgabe zugrunde, eine Schlauchverbindung der eingangs genannten Art zu schaffen, die auch bei Verwendung von Endstücken mit nicht genau zueinander passenden Formen und Abmessungen abgedichtet und gegen unbeabsichtigtes Lösen gesichert werden kann.

Zur Lösung dieser Aufgabe ist erfindungsgemäß vorgesehen, daß die beiden Endstücke einander im wesentlichen angepaßte konische Außen- und Innenflächen aufweisen und daß die Lasche mit einer Spannvorrichtung versehen ist, die an unterschiedliche Durchmesser des anderen Endstückes anpaßbar ist.

Bei der erfindungsgemäßen Schlauchverbindung rastet die Spannvorrichtung an demjenigen Endstück, an dem sie nicht bleibend befestigt ist, nicht etwa in einer bestimmten Nut oder Kerbe ein, sondern die Spannvorrichtung umgreift dieses Endstück ganz oder teilweise, so daß die gegenseitige axiale Zuordnung der beiden Endstücke von den ineinandergesteckten konischen Abschnitten bestimmt wird. Die wirksame Länge der Lasche kann durch die Spannvorrichtung so verändert werden, daß die konischen Abschnitte in der jeweils eingenommenen Stellung durch die Lasche verriegelt werden. Da die Spannvorrichtung an der konischen Außenseite des einen Endstückes klemmend angreift, stellt sich beim Spannen der Spannvorrichtung die wirksame Länge der Lasche stets auf die gegenseitige Zuordnung der Endstücke im ineinandergedrückten Zustand ein. Es ist daher auch möglich, Endstücke mit voneinander abweichenden Kegelmaßen zu verwenden, ohne daß die Gefahr von Undichtigkeiten oder die Gefahr des Lösens bei Zugbelastung besteht. Die Schlauchverbindung kann also auch mit solchen Endstücken realisiert werden, die eigentlich nicht genau aufeinander abgestimmt sind und die nur im wesentlichen einander angepaßt sind, d. h. überhaupt ineinandergeschoben werden können.

Die erfindungsgemäße Schlauchvorrichtung eignet sich insbesondere für die Verbindung zwischen Blasenkathetern und Urinsammelsystemen. Da hier im Falle des Lösens der Schlauchverbindung die Infektionsgefahr besonders groß ist, erfordert dieses Anwendungsgebiet ein hohes Maß an Sicherheit und Dichtigkeit der Schlauchverbindung.

Vorzugsweise weist das zweite Endstück an seinem rückwärtigen Ende eine Ringschulter zum Abstützen der Halterung auf. Dies stellt eine einfache Art der Abstützung dar, bei der die von der Lasche übertragenden Zugkräfte gleichmäßig auf das zweite Endstück übertragen werden.

Bei einer bevorzugten Ausführungsform der Erfindung weist die Halterung einen an der Ringschulter abgestützten Ring und mehrere von dem Ring in Längsrichtung abstehende Laschen auf, deren Stärke sich mit zunehmendem Abstand vom Ring vergrößert, und der Ring ist von einem Schiebering umgeben, der über die Laschen schiebbar ist und diese mit zunehmendem Abstand von dem Ring fester gegen das erste Endstück preßt. Die Sicherheit der Klemmverbindung wird noch dadurch erhöht, daß das erste Endstück eine konische Außenfläche hat, deren Konuswinkel demjenigen der Laschen entgegengesetzt ist. Die Laschen sind vorzugsweise gleichmäßig über den Umfang der Schlauchverbindung verteilt, so daß sich auch die Zugspannungen umfangsmäßig verteilen.

Sollen wesentliche radiale Pressungen der Laschen vermieden werden, kann gemäß einer anderen Alternative die Halterung einen an der Ringschulter abgestützten Ring und mehrere von dem Ring in Längsrichtung abstehende Laschen aufweisen, deren Innenseiten stufenförmig abgesetzt sind und mit dem Stufenbereich das rückwärtige Ende des ersten Endstückes hintergreifen. In diesem Fall erfolgt beim Aufschieben des Schieberinges keine Pressung der Laschen zwischen Schiebering und erstem Endstück, sondern eine Verriegelung der Laschen an dem Endstück. Der Schiebering kann daher ohne großen Kraftaufwand in die Verriegelungsposition gebracht und auch wieder aus dieser gelöst werden.

Wie schon erwähnt, ist es in besonderen Fällen zweckmäßig, Schlauchverbindungen zu benutzen, die nicht einmal durch das Pflegepersonal gelöst werden können. Um dies zu erreichen, kann gemäß einer weiteren Variante der Erfindung vorgesehen sein, daß bei Verwendung von Laschen, deren Stärke sich mit zunehmendem Abstand von dem Ring vergrößert, die Außenseiten der Laschen sägezahnförmig abgestuft sind. Der Schiebering rastet mit zunehmender Verschiebebewegung hintereinander in den einzelnen Sägezähnen der Laschen ein. Da die Sägezähne hinterschnitten sind und die Außenseite der Lasche gegen die Innenseite des Schieberinges drückt, ist es unter normalen Umständen nicht möglich, den Schiebering auf den Laschen zurückzuschieben. Die sägezahnförmigen Laschen bilden zusammen mit dem Schiebering eine Art Ratsche, die nur eine Bewegung des Schieberinges in einer Richtung zuläßt.

Das Spannen der Schlauchverbindung muß nicht notwendigerweise durch axiales Verschieben eines Schieberinges erfolgen, sondern kann auch durch radiales Zusammendrücken zweier die Halterung bildender Teile geschehen. Dabei können zwei einander gegenüberliegende Laschen vorgesehen sein, an deren Enden sich zusammenwirkende Verriegelungselemente befinden, die im Eingriffszustand die konische Außenfläche des ersten Endstückes eng umschließen. Hierbei muß sichergestellt sein, daß die Verriegelungselemente, die durch gegenseitige Radialbewegung in Eingriff miteinander gebracht werden und das erste Endstück fest umschließen, sich nicht unbeabsichtigt wieder auseinanderbewegen können. Zu diesem Zweck können die Verriegelungselemente zusammenwirkende Zacken aufweisen oder eines der Verriegelungselemente weist Zapfen auf, die mit Übermaß in Bohrungen des anderen Verriegelungselementes eindrückbar sind.

Gemäß einer weiteren Variante der Erfindung besteht die Spannvorrichtung aus einem Ringstück, das auf der konischen Außenfläche des ersten Endstückes sitzt und mit der Lasche verbunden ist, und die Halterung weist ein Eingriffselement auf, das eines von mehreren Rastelementen der Lasche umgreift. Diese Ausführungsform ist besonders kostengünstig herstellbar. Die Rastelemente können beispielsweise Rastzähne oder Löcher sein. Das Ringstück ist vorzugsweise C-förmig ausgebildet und umschließt das erste Endstück nur teilweise und elastisch.

Ein besonderer Vorteil der Erfindung besteht darin, daß im Falle einer geringfügigen axialen Verschiebung der Endstücke bei Zugbelastung die an dem ersten Endstück angreifende Spannvorrichtung gespannt wird und ihre Haltekraft bzw. Klemmkraft noch verstärkt. Die Spannvorrichtung braucht also nicht bis an die Grenzen ihrer Belastbarkeit vorgespannt zu werden, was ohnehin zu schnellen Materialermüdungen führen würde, sondern im Belastungsfall stellt sich ihre Spannung entsprechend den jeweiligen Erfordernissen ein. Die Grenze der Belastbarkeit der Schlauchverbindung wird ausschließlich durch die Festigkeit der verschiedenen Materialien bestimmt. Allein durch Zugbeanspruchung der Schläuche kann die Schlauchverbindung nicht zerstörungsfrei gelöst werden.

Im folgenden werden Ausführungsbeispiele der Erfindung unter Bezugnahme auf die Zeichnung näher erläutert. Es zeigt

Fig. 1 einen Längsschnitt durch eine bevorzugte Ausführungsform der Schlauchverbindung,

Fig. 2 einen Längsschnitt durch eine zweite Ausführungsform mit stufenförmig abgesetzten Laschen,

Fig. 3 einen Längsschnitt durch eine gegenüber Fig. 1 geringfügig abgewandelte Ausführungsform, bei der die Spannvorrichtung nach dem Festziehen nicht mehr gelöst werden kann,

Fig. 4 einen Längsschnitt durch eine vierte Ausführungsform der Schlauchverbindung mit radial bewegbaren Verriegelungselementen,

Fig. 5 einen Schnitt entlang der Linie V-V der Fig. 4,

Fig. 6 einen Schnitt ähnlich demjenigen der Fig. 5 durch eine weitere Variante des Ausführungsbeispiels von Fig. 4,

Fig. 7 ein Ausführungsbeispiel, bei dem die Lasche die Form einer Zahnstange hat,

Fig. 8 eine Ansicht des Ausführungsbeispiels der Fig. 7 aus Richtung des Pfeiles VIII und

Fig. 9 ein ähnliches Ausführungsbeispiel wie Fig. 7, jedoch mit einer Lasche in Form eines Lochbandes.

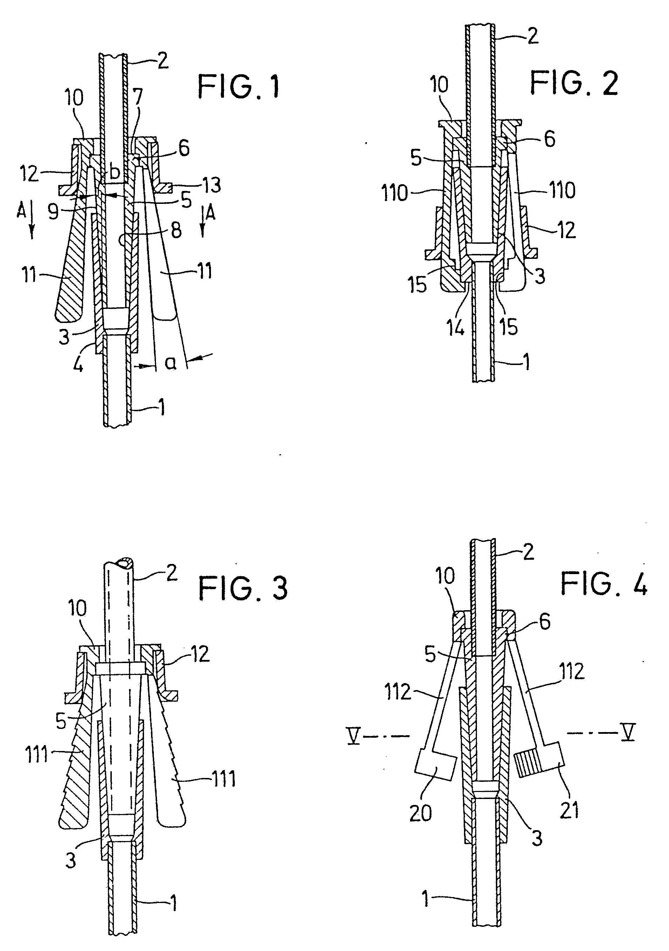

Bei dem Ausführungsbeispiel der Fig. 1 werden durch die Schlauchverbindung die beiden Schläuche 1 und 2 miteinander verbunden. Am Ende des Schlauches 1 ist ein im wesentlichen rohrförmiges Endstück 3 angebracht, dessen Innenfläche sich zum äußeren Ende hin konisch erweitert. Der Konuswinkel ist mit b bezeichnet. Auch die Umfangsfläche 4 des ersten Endstücks 3 erweitert sich geringfügig nach außen hin. Das Ende des Schlauches 1 ist nachträglich in das erste Endstück 3 eingesetzt und mit diesem durch Schweißung oder Kleben fest und abdichtend verbunden.

Das zweite Endstück 5 ist in derselben Weise an dem Schlauch 2 befestigt wie das erste Endstück 3 an dem Schlauch 1. Im Anschluß an den Schlauch 2 ist ein Wulst 6 mit einer rückwärtigen Ringschulter 7 vorgesehen. Von dem Wulst 6 steht nach vorne das im wesentlichen rohrförmige Endstück 5 ab, dessen zylindrische Bohrung 8 der Innenweite des Schlauches 2 entspricht und dessen Außenfläche 9 sich zum vorderen Ende hin konisch verjüngt. Der Konuswinkel des zweiten Endstückes 5 entspricht im wesentlichen dem Konuswinkel b des ersten Endstückes 3.

Auf den Wulst 6 des zweiten Endstückes 5 ist ein Ring 10 aufgeschoben, der sich an der rückwärtigen Ringschulter 7 des Wulstes abstützt. Der Ring 10 weist nach vorne vorstehende Laschen 11 auf, die das erste Endstück 3 teilweise überragen und gleichmäßig über den Umfang verteilt angeordnet sind.

Die Stärke der Laschen 11 nimmt mit zunehmendem Abstand von dem Ring 10 zu, wobei der Steigungswinkel a größer ist als der Kegelwinkel b des ersten Endstückes 3.

Auf dem Ring 10 bzw. den Laschen 11 sitzt koaxial ein Schiebering 12, der an seinem vorderen Ende einen Flansch 13 aufweist. Wird der Schiebering 12 in Richtung der Pfeile A verschoben, dann entsteht nach dem Prinzip der schiefen Ebene bei geringer Verschiebekraft in Pfeilrichtung A eine hohe Radialkraft, wodurch die Laschen 11 zwischen dem ersten Endstück 3 und dem Schiebering 12 eingeklemmt werden. Dadurch entsteht eine Entlastungsverbindung von dem ersten Endstück 3 über die an ihm festgeklemmten Laschen 11 und den Ring 10 zum zweiten Endstück 5. Die Verbindung der ineinandergesteckten Endstücke 3 und 5 wird also über die Laschen 11 gegen Zugkräfte entlastet. Zum Lösen wird der Schiebering 12 in die in Fig. 1 dargestellte Stellung zurückgeschoben.

Das Ausführungsbeispiel der Fig. 2 entspricht weitgehend demjenigen der Fig. 1. Solche Teile, die im wesentlichen identisch sind mit Teilen der Fig. 1 sind mit denselben Bezugszeichen versehen.

Ein wesentlicher Unterschied besteht darin, daß bei der Schlauchverbindung nach Fig. 2 die Laschen 110 keine kontinuierlich sich vergrößernde Stärke aufweisen, sondern die rückwärtige Stirnseite 14 des ersten Endstückes 3 hintergreifen. Die Laschen 110 überragen also in Längsrichtung das erste Endstück 3. An ihren dem Ring 10 abgewandten Enden weisen sie stufenförmige Absätze 15 auf, die im verriegelten Zustand, der in Fig. 2 dargestellt ist, die Stirnflächen 14 hintergreifen. Wird der Schiebering 12 auf den Ring 10 zurückgeschoben, dann federn die Laschen 110 nach außen, so daß sie das erste Endstück 3 wieder freigeben.

Das Ausführungsbeispiel der Fig. 3 entspricht weitgehend demjenigen der Fig. 1. Der einzige Unterschied besteht darin, daß die zu ihren freien Enden hin dicker werdenden Laschen 111 an ihren Außenseiten sägezahnförmig abgestuft sind. Die Sägezähne bilden Hinterschneidungen, in denen die rückwärtige Kante des Schieberinges 12 einrastet. Ein Zurückschieben des Schieberinges 12 ist wegen der Rastwirkung der Sägezähne nicht möglich.

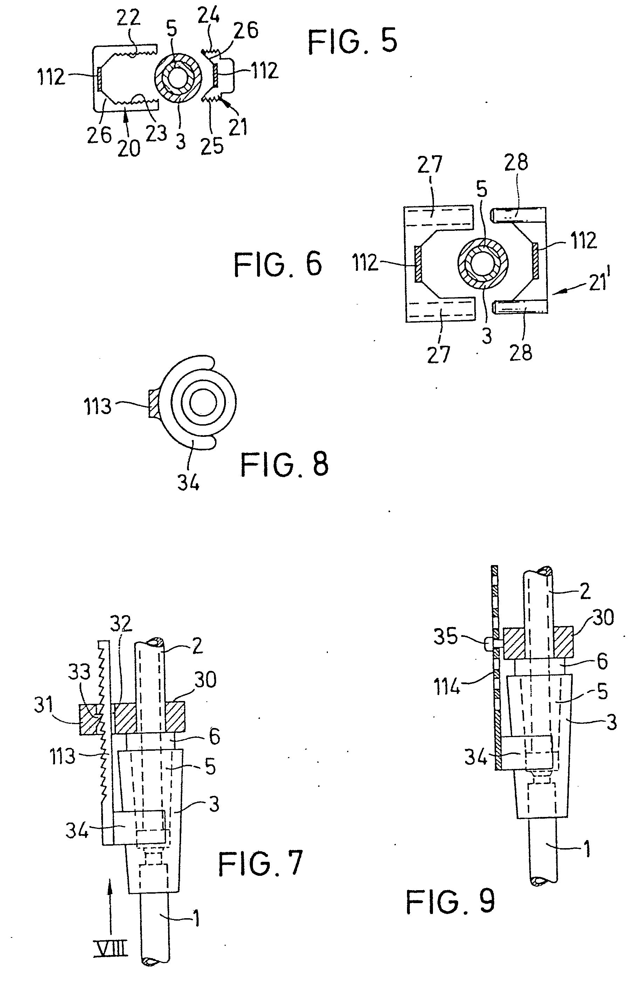

Fig. 4 und 5 zeigen eine besonders kostengünstig herstellbare Variante der Schlauchverbindung, bei der die Verriegelungsvorrichtung aus einem einstückig geformten Kunststoffteil hergestellt werden kann.

Die Verriegelungsvorrichtung besteht aus einem Ring 10, von dem zwei Laschen 112 abstehen, an deren Enden sich jeweils ein Verriegelungselement 20, 21 befindet. Der Ring 10 ist wie bei den vorherigen Ausführungsbeispielen an dem Wulst 6 des zweiten Endstückes 5 abgestützt. Das zweite Endstück 5 ist in das erste Endstück 3 eingesteckt. Die Form der Verriegelungselemente 20, 21 ist aus Fig. 5 ersichtlich. Das Verriegelungselement 20 ist U-förmig und an seinen Innenseiten befinden sich Verzahnungen 22, 23. Das Verriegelungselement 21, das an dem Ende der gegenüberliegenden Lasche 112 angebracht ist, weist entgegengesetzt gerichtete Außenverzahnungen 24, 25 auf. Das U-förmige Verriegelungselement 20 umgreift das erste Endstück 3, während das Verriegelungselement 21 in das Verriegelungselement 20 hineingeschoben wird. Das Endstück 3 wird dabei zwischen dem Endstück 5 und den dreieckigen Rippen 26 der Verriegelungsstücke 20 und 21 eingeklemmt und gesichert. Da der Außenumfang des Endstückes 3 zum äußeren Ende hin ansteigt, ist ein axiales Abziehen der fest ineinandergedrückten Verriegelungselemente 20 und 21 von dem Endstück 3 nicht möglich.

Die Verriegelungselemente 20' und 21', die in Fig. 6 dargestellt sind, greifen im Preßsitz ineinander. Das Verriegelungselement 20' weist zwei parallele Bohrungen 27 auf, in die Zapfen 28 des anderen Verriegelungselementes 21' hineingedrückt werden. Die Durchmesser der Zapfen 28 sind größer als die Durchmesser der Bohrungen 27, so daß die Verriegelungsteile 20' und 21' nach dem Zusammendrücken relativ zueinander festgehalten werden.

Bei dem Ausführungsbeispiel der Fig. 7 ist das Schlauchende 1 ebenfalls fest mit dem ersten Endstück 2 verbunden, in das das mit dem Schlauchende 2 verbundene zweite Schlauchstück 5 eingeschoben ist. An dem Wulst 6 des zweiten Schlauchstücks 5 stützt sich von der Rückseite her ein das Schlauchende 2 umschließender Ring 30 ab, der einen seitlichen Ansatz 31 mit einer Öffnung 32 aufweist, in der sich ein Rastzahn 33 befindet. Die die Ayialkräfte übertragende Lasche 113 besteht aus einer Zahnstange, die durch die Öffnung 32 hindurchragt und mit dem Rastzahn 33 in Eingriff ist. Das untere Ende der Zahnstange ist mit einem seitlich abstehenden Ringstück 34 verbunden, das das Endstück 3 teilweise umschließt. Das Ringstück 34 umschließt, wie Fig. 8 zeigt, einen Winkel von etwas mehr als 180°. Seine Öffnung ist so groß, daß das Ringstück 34 radial über den Schlauch 1 geschoben werden kann, um anschließend auf das konische Endstück 3 aufgeschoben zu werden, bis es fest auf diesem aufsitzt. In dieser Stellung wird das Ringstück 34 durch die Lasche 113 bzw. den in die Verzahnung eingreifenden Rastzahn 33 festgehalten, so daß die Schlauchverbindung gegen axiales Auseinanderziehen gesichert ist. Zum Lösen der Schlauchverbindung wird die Lasche 113 seitlich aus der Öffnung 32 herausgedrückt.

Bei dem Ausführungsbeispiel der Fig. 9 weist der Ring 30 einen seitlich abstehenden Zapfen 35 mit verdicktem Kopf auf. Die Lasche 114 ist ein Lochband, dessen eines Ende mit dem Ringstück 34 verbunden ist und das in Längsrichtung hintereinander zahlreiche Löcher aufweist. Nach dem Aufschieben des Ringstücks 34 auf das Endstück 3 wird das vor dem Zapfen 35 liegende Loch auf den Zapfen 35 aufgedrückt.

## Patentansprüche

1. Schlauchverbindung für medizinische Geräte, mit einem an dem einen Schlauchende (1) befestigten ersten Endstück (3) und einem an dem zweiten Schlauchende (2) befestigten zweiten Endstück (5), das mit dem ersten Endstück (3) durch Einschieben abdichtend verbindbar ist, und einer an einem der Endstücke (3, 5) angreifenden Halterung (10, 30), die über mindestens eine längslaufende Lasche (11, 110, 111, 112, 113, 114) das andere Endstück (3, 5) festhält, dadurch gekennzeichnet, daß die beiden Endstücke (3, 5) einander im wesentlichen angepaßte konische Außen- und Innenflächen aufweisen und daß die Lasche (11, 110, 111, 112, 113, 114) mit einer Spannvorrichtung (11, 12; 20, 21; 34) versehen ist, die an unterschiedliche Durchmesser des anderen Endstückes anpaßbar ist.

2. Schlauchverbindung nach Anspruch 1, dadurch gekennzeichnet, daß das zweite Endstück (5) an seinem rückwärtigen Ende eine Ringschulter (7) zum Abstützen der Halterung (10, 30) aufweist.

3. Schlauchverbindung nach Anspruch 1 oder 2, dadurch gekennzeichnet, daß die Halterung einen an der Ringschulter (7) abgestützten Ring (10) und mehrere von dem Ring (10) in Längsrichtung abstehende Laschen (11) aufweist, deren Stärke sich mit zunehmendem Abstand vom Ring (10) vergrößert und daß der Ring (10) von einem Schiebering (12) umgeben ist, der über die Laschen (11) schiebbar ist und diese mit zunehmendem Abstand von dem Ring fester gegen das erste Endstück (3) preßt.

4. Schlauchverbindung nach Anspruch 3, dadurch gekennzeichnet, daß die Außenseite der Lasche (111) sägezahnförmig abgestuft ist.

5. Schlauchverbindung nach Anspruch 1 oder 2, dadurch gekennzeichnet, daß die Halterung einen an der Ringschulter (7) abgestützten Ring (10) und mehrere von dem Ring (10) in Längsrichtung abstehende Laschen (110) aufweist, deren Innenseiten stufenförmig abgesetzt sind und mit dem Stufenbereich (15) das rückwärtige Ende des ersten Endstückes (3) hintergreifen.

6. Schlauchverbindung nach Anspruch 1 oder 2, dadurch gekennzeichnet, daß zwei gegenüberliegende Laschen (112) vorgesehen sind, an deren Enden sich zusammenwirkende Verriegelungselemente (20, 21; 20', 21') befinden, die im Eingriffszustand die konische Außenfläche des ersten Endstückes (3) eng umschließen.

7. Schlauchverbindung nach Anspruch 6, dadurch gekennzeichnet, daß die Verriegelungselemente (20, 21) zusammenwirkende Zacken (22, 24) aufweisen.

8. Schlauchverbindung nach Anspruch 6, dadurch gekennzeichnet, daß eines der Verriegelungselemente (20', 21') Zapfen (28) aufweist, die mit Übermaß in Bohrungen (27) des anderen Verriegelungselementes (20') eindrückbar sind.

9. Schlauchverbindung nach Anspruch 1, dadurch gekennzeichnet, daß die Spannvorrichtung aus einem Ringstück (34) besteht, das auf der konischen Außenfläche des ersten Endstückes (3) sitzt und mit der Lasche (113, 114) verbunden ist, und daß die Halterung (30) ein Eingriffselement (33, 35) aufweist, das in eines von mehreren Rastelementen der Lasche (113, 114) eingreift.

10. Schlauchverbindung nach Anspruch 9, dadurch gekennzeichnet, daß die Rastelemente Rastzähne sind.

11. Schlauchverbindung nach Anspruch 9, dadurch gekennzeichnet, daß die Rastelemente Löcher sind.

12. Schlauchverbindung nach Anspruch 9, dadurch gekennzeichnet, daß das Ringstück (34) C-förmig ausgebildet ist und das erste Endstück (3) nur teilweise elastisch umschließt.

## Claims

1. Tube joint for medical applicances comprising a first end portion (3) secured to the one tube end (1) and a second end portion (5) secured to the second tube end (2) and being sealingly connectible by insertion to the first end portion (3), a holding device (10, 30) engaging one of the end portions (3, 5) and retaining the other end portion (3, 5) via at least one longitudinally extending flap (11, 110, 111, 112, 113, 114), characterized in that the two end portions (3, 5) have conical external and internal surfaces substantially adapted to one another and that the flap (11, 110, 111, 112, 113, 114) is provided with a clamping means (11, 12; 20, 21; 34) which is adaptable to different diameters of the other end portion.

2. Tube joint according to claim 1, characterized in that the second end portion (5) contains an annular shoulder (7) at its rearward end to support the holding device (10, 30).

3. Tube joint according to claims 1 or 2, characterized in that the holding device contains a ring (10) supported at the annular shoulder (7) and several flaps (11) projecting in longitudinal direction from the ring (10), and the thickness of which grows with the increasing distance from the ring (10), the ring (10) being enclosed by a sliding ring (12) which can be slipped over the flaps (11) to press them more strongly against the first end portion (3) with the increasing distance from the ring.

4. Tube joint according to claim 3, characterized in that the outside of the flap (111) is of a stepped saw-tooth-type shape.

5. Tube joint according to claims 1 or 2, characterized in that the holding device includes a ring (10) supported at the annular shoulder (7) and several flaps (110) projecting in longitudinal direction and whose internal surfaces are offset stepwise, the stepped portion (15) engaging the rearward end of the first end portion (3).

6. Tube joint according to claims 1 or 2, characterized in that two opposite flaps (112) are provided having at their ends cooperating locking elements (20, 21; 20', 21') which, in engagement, closely surround the external surface of the first end portion (3).

7. Tube joint according to claim 6, characterized in that the locking elements (20, 21) contain interacting serrations (22, 24).

8. Tube joint according to claim 6, characterized in that one of the locking elements (20', 21') contains pins (28) which can be pressed with oversize into bores (27) of the other locking element (20').

9. Tube joint according to claim 1, characterized in that the clamping means consists of an annular piece (34) seated on the conical outer surface of the first end portion (3) and connected to the flap (113, 114) and that the holding device (30) contains an engaging element (33, 35) which engages one of several stop elements of the flap (113, 114).

10. Tube joint according to claim 9, characterized in that the stop elements are teeth.

11. Tube joint according to claim 9, characterized in that the stop elements are holes.

12. Tube joint according to claim 9, characterized in that the annular piece (34) is C-shaped and that the first end portion (3) is enclosed by it elastically but only in part.

## Revendications

1. Raccord de tuyaux pour appareils médicaux, comportant une première partie extrême (3) fixée sur une des extrémités de tuyau (1) et une seconde partie extrême (5) fixée sur la seconde extrémité de tuyau (2) et qui peut être reliée de façon étanche par emmanchement avec la première partie extrême (3), ainsi qu'un support (10, 30) s'accrochant sur une des parties extrêmes (3, 5) et qui maintient l'autre partie extrême (3, 5) — par l'intermédiaire d'au moins une patte étendue longitudinalement (11, 110, 111, 112, 113, 114), caractérisé en ce que les deux parties extrêmes (3, 5) comportent des surfaces coniques extérieure et intérieure adaptées essentiellement l'une avec l'autre et en ce que la patte (11, 110, 111, 112, 113 et 114) est pourvue d'un dispositif de serrage (11, 12; 20, 21; 34), qui peut être adapté à des diamètres différents de l'autre partie extrême.

2. Raccord de tuyaux selon la revendication 1, caractérisé en ce que la seconde partie extrême (5) comporte, à son extrémité arrière, un épaulement annulaire (7) destiné à soutenir le support (10, 30).

3. Raccord de tuyaux selon l'une des revendications 1 ou 2, caractérisé en ce que le support comporte un anneau (10) soutenu par l'épaulement annulaire (7) et plusieurs pattes (11) faisant saillie de l'anneau (10) dans une direction longitudinale et dont l'épaisseur augmente avec la distance d'éloignement par rapport à l'anneau (10) et en ce que l'anneau (10) est entouré par un anneau de poussée (12) qui peut être poussé par l'intermédiaire des pattes (11) et qui applique celles-ci, à mesure que la distance d'espacement par rapport à l'anneau augmente, plus étroitement contre la première partie extrême (3).

4. Raccord de tuyaux selon la revendication 3, caractérisé en ce que le côté extérieur de la patte (111) est pourvu d'un profil cranté en forme de dents de scie.

5. Raccord de tuyaux selon l'une des revendications 1 ou 2, caractérisé en ce que le support comporte un anneau (10) s'appuyant contre l'épaulement annulaire (7) et plusieurs pattes (110) faisant saillie de l'anneau (10) dans une direction longitudinale et dont les côtés intérieurs sont pourvus de profils crantés et viennent s'accrocher avec la zone crantée (5) en arrière de l'extrémité arrière de la première partie extrême (3).

6. Raccord de tuyaux selon l'une des revendications 1 ou 2, caractérisé en ce qu'il est prévu deux pattes (112) placées dans des positions opposées et aux extrémités desquelles sont disposés des éléments de verrouillage coopérants (20, 21; 20', 21') qui entourent étroitement, dans la condition d'accrochage, la surface extérieure conique de la première partie extrême (3).

7. Raccord de tuyaux selon la revendication 6, caractérisé en ce que les éléments de verrouillage (20, 21) comportent des dentelures coopérantes (22, 24).

8. Raccord de tuyaux selon la revendication 6, caractérisé en ce qu'un des éléments de verrouillage (20', 21') comporte des broches (28) qui peuvent être emmanchées avec surdimensionnement dans des trous (27) de l'autre élément de verrouillage (20').

9. Raccord de tuyaux selon la revendication 1, caractérisé en ce que le dispositif de serrage se compose d'une partie annulaire (34) qui est montée sur la surface extérieure conique de la première partie extrême (3) et qui est reliée à la patte (113, 114) et en ce que le support (30) comporte un élément d'accrochage (33, 35) qui s'accroche dans l'un de plusieurs éléments d'arrêt de la patte (113, 114).

10. Raccord de tuyaux selon la revendication 9, caractérisé en ce que les éléments d'arrêt sont des dents d'arrêt.

11. Raccord de tuyaux selon la revendication 9, caractérisé en ce que les éléments d'arrêt sont des trous.

12. Raccord de tuyaux selon la revendication 9, caractérisé en ce que la partie annulaire (34) est agencée en forme de C et entoure la première partie extrême (3) seulement en partie et élastiquement.

FIG. 1

FIG. 2

FIG. 3

FIG. 4

FIG. 5

FIG. 6

FIG. 8

FIG. 7

FIG. 9

11